Europäisches Patentamt

European Patent Office

Office européen des brevets

(11) Veröffentlichungsnummer: **0 480 308 A1**

## (12) EUROPÄISCHE PATENTANMELDUNG

(21) Anmeldenummer: **91116857.3**

(22) Anmeldetag: **02.10.91**

(51) Int. Cl.⁵: **C07C 37/72**, C07C 39/08

(30) Priorität: **08.10.90 DE 4031797**

(43) Veröffentlichungstag der Anmeldung:
**15.04.92 Patentblatt 92/16**

(84) Benannte Vertragsstaaten:
**BE CH DE FR GB IT LI NL**

(71) Anmelder: **HOECHST AKTIENGESELLSCHAFT
Postfach 80 03 20
W-6230 Frankfurt am Main 80(DE)**

(72) Erfinder: **Altenbach, Klaus, Dr.
In den Eichen 27
W-6237 Liederbach(DE)**
Erfinder: **Tetzlaff, Heribert, Dr.
Geisenheimer Strasse 88
W-6000 Frankfurt am Main(DE)**
Erfinder: **Hermann, Kurt
Grunerstrasse 15
W-6270 Idstein(DE)**
Erfinder: **Hempe, Michael
Bleichstrasse 9
W-6234 Hattersheim am Main(DE)**

(54) **Verfahren zur Abtrennung von Resorcin aus Resorcin-Vorkondensaten.**

(57) Die Erfindung betrifft ein Verfahren zur extraktiven Abtrennung von freiem Resorcin aus oligomeren Resorcin-Vorkondensaten durch Lösungsextraktion, dadurch gekennzeichnet, daß die Abtrennung durch eine kontinuierliche Lösungsextraktion unter Verwendung von zwei miteinander nicht mischbaren, flüssigen Phasen vorzugsweise nach dem van Dijck-Verfahren erfolgt.

EP 0 480 308 A1

Resorcin-Formaldehyd-Kondensationsprodukte mit niedrigem mittlerem Molekulargewicht, also Oligomere aus Resorcin und Formaldehyd (sogenannte Resorcin-Vorkondensate), werden unter anderem als Haftvermittler zwischen Textilcord- oder Stahlfasern und Gummi in diese Verstärkungsmittel enthaltenden Gummiartikeln, wie Reifen, Treibriemen, Förderbändern und Gummischläuchen, eingesetzt. Insbesondere aus Umweltgesichtspunkten ist der Einsatz dieser Resorcin-Vorkondensate als Haftvermittler dem des reinen Resorcins vorzuziehen, da ihr Dampfdruck wesentlich geringer als derjenige des Resorcins ist und so ein Absublimieren des Resorcins aus der heißen Gummimischung verhindert wird. Für die technische Anwendung dieser Resorcin-Vorkondensate ist es wünschenswert, daß diese einen geringen Restgehalt an freiem Resorcin besitzen und vorzugsweise auch in fester, wasserfreier Form vorliegen.

Aus der JP-Offenlegungsschrift 82/143 316 ist bereits ein Verfahren zur Herstellung von Resorcin-Vorkondensaten mit relativ geringem Restgehalt an freiem Resorcin bekannt, wobei die anfallende rohe Resorcin-Vorkondensatschmelze zur Abreicherung insbesondere des Resorcins mit Xylol extrahiert wird. Nachteilig ist dabei jedoch, daß die Viskosität der Resorcin-Vorkondensatschmelze mit abnehmendem Resorcingehalt stark zunimmt. Das hat einerseits die Bildung von Verklebungen und Verklumpungen an den Apparaturen und andererseits eine starke Verringerung des Extraktionseffektes zur Folge. Der Erniedrigung der Viskosität durch Temperaturerhöhung sind dabei durch das merkliche Auftreten von Zersetzungsprodukten oberhalb von 110 °C enge Grenzen gesetzt.

Es bestand daher das Bedürfnis nach einem Reinigungsverfahren, daß die Nachteile des Standes der Technik nicht aufweist und das insbesondere die wirksame Verringerung des Gehaltes an freiem Resorcin in Resorcin-Vorkondensaten unter milden Bedingungen und in technisch leicht realisierbarer Weise ermöglicht.

Es wurde nun gefunden, daß dies überraschenderweise durch eine Lösungsextraktion unter bestimmten Bedingungen möglich ist.

Die Erfindung betrifft daher ein Verfahren zur Abtrennung von freiem Resorcin aus oligomeren Resorcin-Vorkondensaten, durch Lösungsextraktion, dadurch gekennzeichnet, daß die Abtrennung durch eine kontinuierliche Lösungsextraktion unter Verwendung von zwei miteinander nicht mischbaren, flüssigen Phasen nach dem (der) van Dijck-Verfahren (-Verteilung) erfolgt.

Daneben bezieht sich die Erfindung auch auf ein Verfahren zur extraktiven Abtrennung von freiem Resorcin aus oligomeren Resorcin-Vorkondensaten, durch Lösungsextraktion, dadurch gekennzeichnet, daß die Abtrennung durch eine kontinuierliche Lösungsextraktion unter Verwendung von Ether, vorzugsweise Diisopropylether und Wasser als Extraktionsphasen erfolgt.

Die zur Reinigung eingesetzten rohen, noch freies Resorcin enthaltenden Resorcin-Vorkondensate können nach beliebigen Methoden hergestellt werden, z.B. nach dem Verfahren der DE-Offenlegungsschrift 40 02 417.2 oder nach herkömmlichen Verfahren in homogener Phase, z.B. beschrieben in A. Knop und L.A. Pilato, "Phenolic Resins", Springer Verlag, Berlin 1985, Seiten 90 bis 300.

Sie enthalten im allgemeinen noch bis zu 50 Gew.-%, vorzugsweise 15 bis 40 Gew.-%, bezogen auf Resorcin-Vorkondensat, an freiem Resorcin. In der Regel werden diese Resorcin-Vorkondensate für den erfindungsgemäßen Reinigungsprozeß in Form von Lösungen eingesetzt, wobei hierfür vorzugsweise Ether, insbesondere Diisopropylether, als Lösungsmittel verwendet werden können.

Das mittlere Molekulargewicht dieser Resorcin-Vorkondensate ist nicht kritisch und kann in dem für Oligomere typischen Bereich schwanken. Vorzugsweise liegt es zwischen ca. 230 und ca. 500, insbesondere zwischen ca. 280 und ca. 420 (Gewichtsmittel).

Als flüssige, miteinander nicht mischbare Phasen kommt erfindungsgemäß vorzugsweise das System Ether/Wasser zum Einsatz, wobei der Ether insgesamt bevorzugt mindestens 4 C-Atome, insbesondere 4 bis 18 C-Atome enthält.

Das Mengenverhältnis zwischen Ether, vorzugsweise Diisopropylether, und Wasser liegt im allgemeinen zwischen 1:3 und 1:20, vorzugsweise zwischen 1:8 und 1:12.

Die erfindungsgemäß bevorzugt verwendete Verfahrensweise nach van Dijck ist beispielsweise beschrieben in Houben-Weyl, Methoden der organischen Chemie, 4. Aufl. (1958), G.Thieme Verlag Stuttgart, Bd. I 1, S. 249-255. Auf diese Literaturstelle wird hiermit Bezug genommen. Wesentlich ist bei dieser Verfahrensweise, daß die zu reinigende Substanz, hier das Resorcin-Vorkondensat, in die Mitte eines mehrstufigen Verteilungsbettes (Extraktionsvorrichtung) eingebracht wird, und daß die beiden flüssigen, nichtmischbaren Phasen an den Enden der Extraktionsvorrichtung eingespeist und im Gegenstrom geführt werden. Auf diese Weise gelingt es an einem Ende der Extraktionsvorrichtung eine wäßrige Resorcinlösung zu gewinnen, die nur geringe Anteile an Resorcin-Vorkondensat aufweist und die beispielsweise wieder zur Herstellung des Vorkondensates verwendet werden kann. Am anderen Ende der Extraktionsvorrichtung wird demgegenüber eine Etherlösung des Resorcin-Vorkondensates erhalten, die zumeist höchstens noch 5 Gew.-% Resorcin enthält. Nach Abdampfen des Ethers, der wieder eingesetzt werden kann, verbleibt eine entsprechende Resorcin-Vorkondensat-Schmelze, die

anschließend vorzugsweise pelletiert wird. Andere grundsätzlich auch erfindungsgemäß einsetzbare Verfahrensführungen der Lösungsextraktion sind gleichfalls in der vorstehend zitierten Literaturstelle beschrieben.

Das erfindungsgemäße Verfahren wird vorzugsweise bei Raumtemperatur durchgeführt, wenngleich auch mäßig erhöhte Temperaturen bis zu etwa 90 °C, speziell bis zu etwa 50 °C, möglich sind. Im allgemeinen wird bei Normaldruck gearbeitet; in Sonderfällen kann aber auch erhöhter oder erniedrigter Druck eingesetzt werden.

Als Apparate für die Extraktion sind die üblichen Extraktionsvorrichtungen, wie Mixer-Settler-Apparaturen, Extraktionskolonnen mit Schwingböden, mit rotierenden oder fest eingebauten Böden, einfach pulsierende Füllkörpersäulen, Mischabsetzer, Graesser-Extraktoren, Zentrifugalextraktoren udgl. geeignet, in denen das Rohgemisch in der Mitte eingespeist wird und die Extraktionsmittel im Gegenstrom geführt werden. Das Verhältnis von Zulaufmenge der Lösung des rohen Resorcin-Vorkondensates zur Zulaufmenge des Ethers beträgt im allgemeinen 1:2 bis 2:1, vorzugsweise 1,5:1 bis 1:1,5. Auch andere Mengenverhältnisse sind möglich; diese können vom Fachmann leicht ermittelt werden und sind abhängig vom gewünschten Extraktionsgrad, der verwendeten Extraktionsmittel und von der Zusammensetzung des Rohgemisches. Die Konzentration des der Extraktionsvorrichtung zugegebenen rohen Resorcin-Vorkondensates in dem Lösungsmittel, vorzugsweise einem der obigen Ether, beträgt zweckmäßigerweise höchstens 50 Gew.-%, vorzugsweise 20 bis 40 Gew.-%.

Das Mengenverhältnis von Vorkondensatlösung:Ether:Wasser liegt erfindungsgemäß zumeist bei 0,5 bis 1,5 : 0,5 bis 1,5 : 8 bis 14, vorzugsweise 0,75 bis 1,25 : 0,75 bis 1,25 : 10 bis 12.

In der Regel reicht eine einmalige Durchführung der Extraktion in der Extraktionsvorrichtung aus, um ein Resorcin-Vorkondensat der gewünschten Reinheit zu erhalten. Die Stufenzahlen betragen dabei im allgemeinen höchstens 10, vorzugsweise 4 bis 6. Im Bedarfsfalle - wenn ein besonders geringer Restgehalt an freiem Resorcin angestrebt wird - kann aber diese Extraktionstrennung, nach vorheriger Isolierung des Resorcin-Vorkondensats und Herstellung einer entsprechend konzentrierten Lösung, wiederholt werden.

Nach einer bevorzugten Ausführungsform des erfindungsgemäßen Verfahrens wird eine Lösung des rohen Resorcin-Vorkondensates in Ether, vorzugsweise Diisopropylether, bei Raumtemperatur in die Mitte einer Gegenstromextraktionskolonne eingepumpt und darin mehrstufig in einem Gegenstrombett aus Wasser und Ether, vorzugsweise Diisopropylether, verteilt. In der Kolonne strömt Wasser als kontinuierliche Phase von oben nach unten, während der Ether als disperse Phase von unten nach oben geführt wird. Das Mengenverhältnis von Ether, vorzugsweise Diisopropylether, zu Wasser liegt hierbei zwischen 1:8 und 1:12. Aus der am oberen Ende ablaufenden Ether (Diisopropylether)-/Resorcin-Vorkondensat-Phase werden die Resorcin-Vorkondensate durch Verdampfung des Ethers isoliert. Geringe, in der Etherphase gelöste Wassermengen gehen dabei als Azeotrop mit dem Ether (Diisopropylether; Sdp. 62,2 °C; 4,5 Gew.-% Wasseranteil) ab. Der Ether (Diisopropylether) wird dann in die Extraktion zurückgeführt. Die am unteren Ende der Extraktionskolonne ablaufende resorcinhaltige Wasserphase wird einer gesonderten Resorcin-Extraktion unterworfen und läuft anschließend ebenfalls im Kreislauf in die Extraktion zurück. Die Resorcin-Extraktion der Wasserphase erfolgt dabei bevorzugt mit Diisopropylether, und die so erhaltene Resorcin/Diisopropylether-Phase wird dann, nach entsprechender Aufstockung des Resorcingehaltes, wieder dem Herstellungsprozeß des Resorcin-Vorkondensates zugeführt.

Der Restgehalt an freiem Resorcin im Resorcin-Vorkondensat kann erfindungsgemäß leicht über das Mengenverhältnis der beiden flüssigen Extraktionsphasen und die Stufenzahl der Extraktion eingestellt werden.

Es war nicht vorhersehbar und ist als überraschend anzusehen, daß mit dem erfindungsgemäßen, wenig aufwendigen Verfahren unter Einsatz spezieller Extraktionsmittel Resorcin-Vorkondensate unter milden Bedingungen und mit geringem, bei ihrem bestimmungsgemäßen Einsatz nicht mehr störenden Gehalt an freiem Resorcin von zumeist kleiner als 5 Gew.-%, vorzugsweise kleiner als 3 Gew.-%, erhältlich sind, zumal Ether, insbesondere der Diisopropylether, als gute Lösungsmittel für Resorcin bekannt waren und daher eine Trennung hiermit nicht zu erwarten war. Die erfindungsgemäß mögliche hohe Trennwirkung war auch deswegen überraschend, als eine Reihe von anderen gebräuchlichen Extraktions-Systemen, wie Toluol/Wasser oder Xylol/Wasser nur unbefriedigende Ergebnisse liefern.

Die erfindungsgemäß gereinigten Resorcin-Vorkondensate eignen sich z.B. in Kombination mit formaldehydabspaltenden Verbindungen in vulkanisierbaren Kautschukmischungen die Verstärkungsmittel auf Textilfaser- oder Stahlcordbasis enthalten, zur Haftverbesserung zwischen Gummi und dem Textil- bzw. Stahlcord in den Vulkanisaten. Durch den, vergleichsweise zu freiem Resorcin, niedrigen Dampfdruck der erfindungsgemäß gereinigten Resorcin-Vorkondensate wird die Abluftbelastung durch toxische Substanzen, wie sie bekanntlich bei der Verwendung von freiem Resorcin durch die erforderlichen hohen Verarbeitungstemperaturen bei der Herstellung vulkanisierbarer Kautschukmischungen auftritt, stark verringert.

Das erfindungsgemäße Verfahren soll anhand der folgenden Beispiele näher erläutert werden:

Beispiel 1

Eine Reaktionslösung von 10,3 Gew.-% Resorcin und 25,4 Gew.-% Resorcin-Vorkondensat, jeweils bezogen auf die Lösung in Diisopropylether wurde bei Raumtemperatur in die Mitte einer Schwingbodenkolonne (2 m Länge, 25 mm Innendurchmesser, 40-Sieb-Böden, Typ Karr) eingespeist. Am unteren Ende der Kolonne wurde Diisopropylether als disperse Phase, am oberen Ende Leitungswasser als kontinuierliche Phase aufgegeben. Das Mengenverhältnis Vorkondensatlösung : Diisopropylether : Wasser betrug 1,03 : 1,0 : 12,75. Die am oberen Ende der Kolonne ablaufende Diisopropyletherphase enthielt 11,25 Gew.-% Resorcin-Vorkondensat und 0,31 Gew.-% Resorcin und die am unteren Ende ablaufende Wasserphase enthielt 0,78 Gew.-% Resorcin und 0,25 Gew.-% Resorcin-Vorkondensat. Die Resorcin-Vorkondensatphase wurde im Dünnschichtverdampfer vom Diisopropylether befreit und die anfallende Schmelze wurde anschließend pelletiert. Der Ether wurde in den Prozeß zurückgeführt.

Beispiel 2

Eine Reaktionslösung von 3,8 Gew.-% Resorcin und 20,95 Gew.-% Resorcin-Vorkondensat, jeweils bezogen auf die Lösung in Diisopropylether wurde bei Raumtemperatur in die Mitte eines 7-stufigen Labormischabsetzers (wie in der DE-Patentanmeldung P 39 13 779.1 beschrieben) eingespeist. Das Mengenverhältnis Vorkondensat-Lösung : Diisopropylether : Wasser betrug 1:1,4:12,6. Die an einem Ende des Mischabsetzers ablaufende Wasserphase enthielt 0,26 Gew.-% Resorcin und 0,19 Gew.-% Resorcin-Vorkondensate, und die am anderen Ende ablaufende Diisopropyletherphase enthielt 0,22 Gew.-% Resorcin und 7,73 Gew.-% Vorkondensate.
Die Aufarbeitung erfolgte wie in Beispiel 1 beschrieben.

Beispiel 3

Eine Reaktionslösung von 9,8 Gew.-% Resorcin und 26,4 Gew.-% Resorcin-Vorkondensat, jeweils bezogen auf die Lösung, in Diisopropylether wurde bei Raumtemperatur in die Mitte einer Technikums-Schwingbodenkolonne (3 m Länge, 50 mm Innendurchmesser, 30 Sieb-Böden, Typ Karr) eingespeist. Am unteren Ende der Kolonne wurde Diisopropylether als disperse Phase, am oberen Ende Leitungswasser als kontinuierliche Phase aufgegeben. Das Mengenverhältnis Reaktionslösung : Diisopropylether : Wasser betrug 1,0 : 1,0 : 10,0. Die am oberen Ende der Kolonne ablaufende Diisopropyletherphase enthielt 13,1 Gew.-% Resorcin-Vorkondensat und 0,08 Gew.-% Resorcin, und die am unteren Ende ablaufende Wasserphase enthielt 0,97 Gew.-% Resorcin und 0,045 Gew.-% Vorkondensat.
Die Aufarbeitung erfolgte wie in Beispiel 1 beschrieben.

**Patentansprüche**

1. Verfahren zur extraktiven Abtrennung von freiem Resorcin aus oligomeren Resorcin-Vorkondensaten durch Lösungsextraktion, dadurch gekennzeichnet, daß die Abtrennung durch eine kontinuierliche Lösungsextraktion unter Verwendung von zwei miteinander nicht mischbaren, flüssigen Phasen nach dem van Dijck-Verfahren erfolgt.

2. Verfahren nach Anspruch 1, dadurch gekennzeichnet, daß als flüssige Phasen ein Ether und Wasser eingesetzt werden.

3. Verfahren nach Anspruch 1 oder 2, dadurch gekennzeichnet, daß das Mengenverhältnis von Ether zu Wasser zwischen 1:3 und 1:20, vorzugsweise 1:8 und 1:12 liegt.

4. Verfahren nach mindestens einem der Ansprüche 1 bis 3, dadurch gekennzeichnet, daß das rohe Resorcin-Vorkondensat in Form einer Lösung eingesetzt wird.

5. Verfahren nach Anspruch 4, dadurch gekennzeichnet, daß als Lösungsmittel Wasser oder ein damit nicht-mischbares organisches Lösungsmittel dient.

6. Verfahren nach Anspruch 4 oder 5, dadurch gekennzeichnet, daß die Lösung des rohen Resorcin-Vorkondensates in der Mitte des Verteilungsbettes (Extraktionsvorrichtung) eingespeist wird, und das Verhältnis an Lösungsmenge zu Menge an Ether zwischen 1:2 und 2:1 liegt.

7. Verfahren nach mindestens einem der Ansprüche 1 bis 6, dadurch gekennzeichnet, daß die Lösungsextraktion bei Raumtemperatur und in einer Extraktionskolonne als Verteilungsbett durchgeführt wird.

8. Verfahren nach mindestens einem der Ansprüche 1 bis 7, dadurch gekennzeichnet, daß die Aufarbeitung der das Verteilungsbett verlassenden Phasen destillativ oder extraktiv erfolgt.

9. Verfahren nach mindestens einem der Ansprüche 1 bis 8, dadurch gekennzeichnet, daß der Reinheitsgrad der das Verteilungsbett verlassenden Lösung des Resorcin-Vorkondensates und der Resorcin-Phase über das Mengenverhältnis der beiden flüssigen Extraktionsphasen und die Stufenzahl der Extraktion eingestellt wird.

10. Verfahren nach Anspruch 2 oder 5, dadurch gekennzeichnet, daß als Ether Diisopropylether eingesetzt wird.

11. Verfahren zur extraktiven Abtrennung von freiem Resorcin aus oligomeren Resorcin-Vorkondensaten, durch Lösungsextraktion, dadurch gekennzeichnet, daß die Abtrennung durch eine kontinuierliche Lösungsextraktion unter Verwendung von Ether und Wasser als Extraktionsphasen erfolgt.

12. Verfahren nach Anspruch 11, dadurch gekennzeichnet, daß als Ether Diisopropylether dient.

**Europäisches Patentamt**

# EUROPÄISCHER RECHERCHENBERICHT

Nummer der Anmeldung

## EP 91 11 6857

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | GB-A-1 308 026 (MITSUI TOATSU CHEMICALS) <br> * Seite 2, Zeile 59 - Seite 2, Zeile 80 * * <br> — — — | 1-12 | C 07 C 37/72 <br> C 07 C 39/08 |
| Y | DE-A-2 261 640 (FARBWERKE HOECHST) <br> * Seite 5, Absatz 3 - Seite 6, Absatz 1 * * <br> — — — | 1-12 | |
| A | US-A-2 245 945 (W.J. VAN DIJCK ET AL.) <br> * Seite 1, Spalte 2, Zeile 49 - Seite 2, Spalte 1, Zeile 27 * * <br> — — — | 1 | |
| A | EP-A-0 000 165 (HAARMAN & REIMER) <br> * Seite 8, Zeile 11 - Seite 8, Zeile 26 * * <br> — — — | 1 | |
| A | US-A-1 762 979 (I. GUBELMANN ET AL) <br> * Seite 1, Zeile 26 - Seite 1, Zeile 70 * * <br> — — — — — | 1 | |

|  | RECHERCHIERTE SACHGEBIETE (Int. Cl.5) |
|---|---|
|  | C 07 C |

**Der vorliegende Recherchenbericht wurde für alle Patentansprüche erstellt**

| Recherchenort | Abschlußdatum der Recherche | Prüfer |
|---|---|---|
| Berlin | 17 Januar 92 | PROBERT C.L. |